# EUROPÄISCHE PATENTSCHRIFT

(11) **EP 0 758 316 B1**
(45) Veröffentlichungstag und Bekanntmachung des Hinweises auf die Patenterteilung: **18.11.1998**
(21) Anmeldenummer: 95915867.6
(22) Anmeldetag: 13.04.1995
(51) Int. Cl.: C07C 271/22, C07D 301/16, A61K 31/27, A61K 31/335

(54) **TUBULIN-POLYMERISATION BZW. -DEPOLYMERISATION BEEINFLUSSENDE BORNEOLDERIVATE**
BORNEOL DERIVATIVES AFFECTING TUBULIN POLYMERIZATION AND DEPOLYMERIZATION
DERIVES DE BORNEOL INFLUANT SUR LA POLYMERISATION ET LA DEPOLYMERISATION DE LA TUBULINE

(30) Priorität: 05.05.1994 DE 4416374
(43) Veröffentlichungstag der Anmeldung: 19.02.1997
(73) Patentinhaber: SCHERING AKTIENGESELLSCHAFT, 13342 Berlin (DE)
(72) Erfinder: KLAR, Ulrich, D-13503 Berlin (DE); GRAF, Hermann, D-13465 Berlin (DE); NEEF, Günter, D-10771 Berlin (DE); BLECHERT, Siegfried, D-13503 Berlin (DE)
(86) Internationale Anmeldenummer: EP9501341
(87) Internationale Veröffentlichungsnummer: WO9530650

(56) Entgegenhaltungen:
- EP-A- 0 253 738
- JOURNAL OF THE AMERICAN CHEMICAL SOCIETY, Bd.114, 1992, WASHINGTON, DC US Seiten 5878 - 79 P. A. WENDER, TH. P. MUCCIARO 'A new and practical approach to the synthesis of taxol and taxol analogues: the pinene path' in der Anmeldung erwähnt

## Beschreibung

Die Erfindung betrifft neue pharmakologisch wirksame Verbindungen, welche die Fähigkeit haben, die Tubulin-Polymerisation bzw. Tubulin-Depolymerisation zu beeinflussen.

Eine Reihe natürlicher Mitosegifte werden als Antitumormittel eingesetzt bzw. befinden sich in der klinischen Prüfung. Es existieren verschiedene Klassen dieser Mitosegifte, die ihre zytotoxische Wirkung entweder durch Inhibition der Polymerisation von Mikrotubuli im Spindelapparat entfalten (z. B. Vinka-Alkaloide, Colchicin) oder dies durch eine GTP-unabhängige Steigerung der Polymerisation des Tubulins und Verhinderung der Depolymerisation von Mikrotubuli erreichen (z. B. Taxol, Taxotere). Mitosegifte haben aufgrund bisher unverstandener physikochemischer Eigenschaften und durch die Besonderheiten neoplastischer Zellen eine gewisse Selektivität für Tumorzellen, es besteht jedoch auch eine nicht unerhebliche Zytotoxizität gegenüber nicht transformierten Zellen.
Vinka-Alkaloide besitzen bis heute große Bedeutung in der kombinierten Chemotherapie myeloischer Tumore. Taxane haben in jüngster Zeit bedeutende Anwendungsgebiete erschlossen, die durch bisher verfügbare Zytostatika nicht zugänglich waren, z.B. Ovarialkarzinome, maligne Melanome. Die Nebenwirkungen der Taxane sind allerdings denen anderer Zytostatika vergleichbar (z.B. Haarausfall, sensorische Neuropathie). Multi-Drug-resistente Tumorzellen, die das P-Glycoprotein überexprimieren sind gegenüber Taxanen resistent. Die begrenzte Verfügbarkeit des Naturstoffs Taxol behindert zudem eine breitere klinische Testung.
Es wurden deshalb Naturstoffe und synthetische Pharmaka getestet, die ein von den bisherigen Mitosegiften abweichendes Wirkspektrum besitzen. Eine *in vitro* Versuchsanordnung ermöglicht die Suche nach Substanzen, die die GTP-abhängige Polymersiation von Tubulin nicht beeinflussen, die Depolymersiation der gebildeten Mikrotubuli jedoch verhindern. Substanzen mit einem solchen Wirkprofil sollten die vielseitigen Funktionen von Mikrotubuli in extranukleären Zellkompartimenten weniger stark beeinflussen, als die Dynamik des Spindelapparats während der Mitose (Meta/Anaphase). Folgerichtig sollten solche Verbindungen geringere Nebenwirkungen *in vivo* zeigen als Taxane oder Vinka-Alkaloide.
Tubulin ist ein essentieller Bestandteil der mitotischen Spindel. Es dient unter anderem zur Aufrechterhaltung der Zellform, zum Transport von Organellen innerhalb der Zelle und zur Beeinflussung der Zellbeweglichkeit.
Taxane stellten bislang die einzige bekannte Strukturklasse dar, die in der Lage ist, die Polymerisation von Tubulin (überwiegend in der G2-Phase) zu beschleunigen sowie die gebildeten Mikrotubuli-Polymere zu stabilisieren. Dieser Mechanismus unterscheidet sich deutlich von denjenigen die andere, ebenfalls die phasenspezifische Zellteilung beeinflussende Strukturklassen aufweisen. So hemmen beispielsweise Substanzen aus der Gruppe der Vinka-Alkaloide (z.B. Vincristine und Vinblastine) aber auch Colchicin die Polymerisation der Tubulindimeren in der M-Phase.
Es wurde nun gefunden, daß vergleichsweise einfach herzustellende Verbindungen der Formel I in der Lage sind, die Depolymerisation von Mikrotubuli zu hemmen, ohne GTP-unabhängig die Bildung von Mikrotubuli zu steigern. Darüber hinaus wurden Verbindungen mit einem völlig neuen Wirkprofil identifiziert, die in der Lage sind, die Depolymerisation von Mikrotubuli zu beschleunigen. Auf Grund dieser Eigenschaften stellen die Verbindungen der Formel I wertvolle Pharmaka dar, die prinzipiell in der Lage sind, die schwer zu synthetisierenden und in ausreichenden Mengen noch nicht verfügbaren Taxane wie z.B. Taxol und Taxotere® in der Therapie maligner Tumore zu ergänzen bzw. zu ersetzen (EP-A 253739).
Die neuen Borneolderivate sind gekennzeichnet durch die allgemeine Formel I 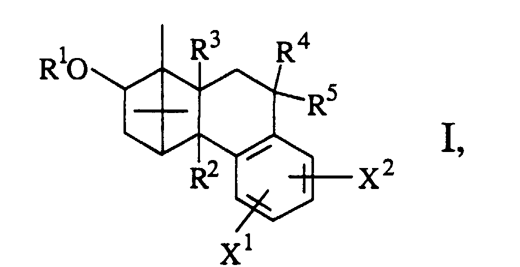 worin
- R¹: C(O)-CH(OR⁶)-CH(NHR⁷)-R⁸,
- R²: Wasserstoff, -OH, C₁-C₁₀-Alkyl, C₁-C₁₀-Alkoxy, -OC(O)R^{9a}, -OSO₂R^{9a}, -OP(O)(OH)₂, NHR^{9a}, NR^{9a}R^{9b},
- R³: Wasserstoff, -OH, C₁-C₁₀-Alkoxy, -OC(O)R^{9b}, -OSO₂R^{9b}, -OP(O)(OH)₂, oder
- R², R³: gemeinsam ein Sauerstoffatom,
- R⁴: Wasserstoff, C₁-C₁₀-Alkyl, -(CH₂)ₙ-OR^{11a},
- R⁵: Wasserstoff, C₁-C₁₀-Alkyl, -(CH₂)ₚ-OR^{11b}, oder
- R⁴, R⁵: gemeinsam ein Sauerstoffatom, eine =CHR¹⁰-Gruppe,
- n: 0 bis 8
- p: 1 bis 8
- R⁷: -C(O)R¹², -SO₂R¹², -C(O)OR¹², -C(O)NHR^{9d}, -C(O)NR^{9d}R^{9e}, 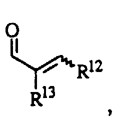
- R⁸: Aryl,
- R^{9a-e}, R¹²: gleich oder verschieden sind und C₁-C₁₀-Alkyl, C₄-C₈-Cycloalkyl, Aryl, C₇-C₁₆-Aralkyl bedeuten,
- R¹⁰: Wasserstoff, C₁-C₁₀-Alkyl, -(CH₂)_{S}-OR¹⁴,
- s: 1 bis 8
- R⁶, R^{11a,b}, R¹⁴: gleich oder verschieden sind und Wasserstoff, C₁-C₁₀-Alkyl, Aryl, C₇-C₁₆-Aralkyl, -SO₂R^{9c}, -P(O)(OH)₂ bedeuten,
- R¹³, R^{15a,b}: gleich oder verschieden sind und Wasserstoff, C₁-C₁₀-Alkyl, Aryl, C₇-C₁₆-Aralkyl bedeuten,
- X¹, X²: gleich oder verschieden sind und X bedeuten,
- X: Wasserstoff, Halogen, -OH, -NO₂, -N₃, -CN, -NR^{15a}R^{15b}, -NHSO₂R^{15a}, -CO₂R¹⁵, C₁-C₁₀-Alkyl, C₁-C₁₀-Alkoxy, C₁-C₁₀-Acyloxy, C₁-C₁₀-Acyl sein kann
und, falls R¹⁵ Wasserstoff bedeutet, deren Salze mit physiologisch verträglichen Basen, sowie deren α-, β- oder γ-Cyclodextrinclathrate, sowie die mit Liposomen verkapselten Verbindungen der allgemeinen Formel I bedeuten.

Die Erfindung betrifft die Diastereomeren und/oder Enantiomeren dieser Borneolderivate und auch deren Gemische.
Als Alkylgruppen R², R⁴, R⁵, R⁶, R^{9a-e}, R¹⁰, R^{11a,b}, R¹², R¹³, R¹⁴, R^{15a,b} und X sind gerad- oder verzweigtkettige Alkylgruppen mit 1-10 Kohlenstoffatomen zu betrachten, wie beispielsweise Methyl, Ethyl, Propyl, Isopropyl, Butyl, Isobutyl, tert.-Butyl, Pentyl, Isopentyl, Neopentyl, Heptyl, Hexyl, Decyl.
Die Alkylgruppen R², R⁴, R⁵, R⁶, R^{9a-e}, R¹⁰, R^{11a,b}, R¹², R¹³, R¹⁴, R^{15a,b} und X können substituiert sein durch 1-3 Halogenatome, Hydroxygruppen, C₁-C₄-Alkoxygruppen, C₆-C₁₂-Arylgruppen, die durch 1-3 Halogenatome substituiert sein können, Di-(C₁-C₄)-Alkylamine und Tri-(C₁-C₄)-Alkylammonium.
Als Cycloalkylgruppen R^{9a-e}, R¹² kommen substituierte und unsubstituierte Reste mit 4 bis 8 Kohlenstoffatomen in Betracht.
Als Arylrest R⁶, R⁸ ,R^{9a-e}, R¹⁰, R^{11a,b}, R¹², R¹³, R¹⁴, R^{15a,b} kommen substituierte und unsubstituierte carbocyclische oder heterocyclische Reste wie z.B. Phenyl, Naphtyl, Furyl, Thienyl, Pyridyl, Pyrazolyl, Pyrimidinyl, Oxazolyl, Pyridazinyl, Pyrazinyl, Chinolyl, die mehrfach substituiert sein können durch die in X definierten Gruppen, in Frage.
Die in R², R³ und X der allgemeinen Formel I enthaltenen Alkoxy-, Acyl- sowie Acyloxygruppen sollen jeweils 1 bis 10 Kohlenstoffatome enthalten, wobei Methoxy-, Ethoxy- Propoxy- Isopropoxy-, t-Butyloxy-, Formyl, Acetyl, Propionyl-. und Isopropionylgruppen bevorzugt sind.
Die C₇-C₁₆-Aralkylgruppen in R⁶, R^{9a-e}, R^{11a,b}, R¹², R¹³, R¹⁴, R^{15a,b} können im Ring bis 14 C-Atome enthalten, bevorzugt 6 bis 10 und in der Alkylkette 1 bis 4 , bevorzugt 1 bis 2 Atome. Bevorzugte Aralkylreste sind z.B. Benzyl, Phenylethyl, Naphtylmethyl bzw. Naphtylethyl. Die Ringe können mehrfach substituiert sein durch die in X definierten Gruppen.
Freie Hydroxygruppen in R¹, R², R³, R⁴, R⁵, R¹⁰ und X können funktionell abgewandelt sein, beispielsweise durch Veretherung oder Veresterung, wobei freie Hydroxygruppen bevorzugt sind.
Als Ether- und Acylreste kommen die dem Fachmann bekannten Reste in Betracht. Bevorzugt sind leicht abspaltbare Etherreste, wie beispielsweise der Tetrahydropyranyl-, Tetrahydrofuranyl-, tert.-Butyldimethylsilyl-, tert.-Butyldiphenylsilyl-, Tribenzylsilylrest. Als Acylreste kommen z.B. Acetyl, Propionyl, Butyryl, Benzoyl in Frage.
Halogen in den Definitionen für X bedeutet Fluor, Chlor, Brom und Iod.
Zur Salzbildung mit den freien Säuren (R¹⁵ = H) sind anorganische und organische Basen geeignet, wie sie dem Fachmann zur Bildung physiologisch verträglicher Salze bekannt sind. Beispielsweise seien genannt: Alkalihydroxide, wie Natrium- oder Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin u.s.w.

Die Erfindung betrifft ferner Verfahren zur Herstellung von Borneolderivaten der Formel I, welches dadurch gekennzeichnet, daß man ein Olefin der allgemeinen Formel II 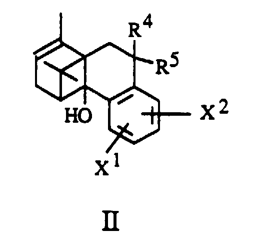 in dem R⁴, R⁵, X¹ und X² die oben genannten Bedeutungen haben und in X¹ oder X² enthaltene Hydroxylgruppen gegebenfalls geschützt sind, epoxidiert und das gebildete Epoxid ohne Isolation zu einem Alkohol der allgemeinen Formel III 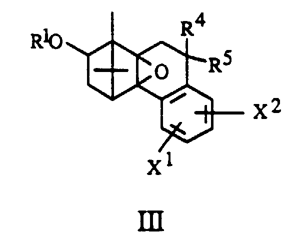 in dem R⁴, R⁵, X¹ und X² die oben genannten Bedeutungen haben und in R¹, X¹ oder X² enthaltene Hydroxylgruppen gegebenfalls geschützt sind, umlagert und dieses Umlagerungsprodukt in ein Derivat der allgemeinen Formel I übeführt.

Die Reaktionsbedingungen der vorgenannten Verfahrensstufen sind:
a) II → III
   Die Epoxidierung der Doppelbindung erfolgt mit einer Peroxyverbindung wie z.B. meta-Chlorperbenzoesäure, Peroxotrifluoressigsäure, Wasserstoffperoxid, tert.-Butylhydroperoxid gegebenfalls unter Zusatz einer Lewissäure wie z.B. Titantetraisopropoxid in einem inerten Solvens wie z.B. Dichlormethan, Toluol bei -40°C bis +40°C. Bevorzugt ist die Umsetzung mit tert.-Butylhydroperoxid und Titantetraisopropoxid in Toluol bei - 10°C bis + 25°C.
   Die Umlagerung des gebildeten Epoxides wird durch Säuren wie z.B. para-Toluolsulfonsäure, Kieselgel, saure Ionenaustauscherharze, Salzsäure katalysiert. Bevorzugt ist die Verwendung von Kieselgel.
b) III → I
   Die Überführung der Verbindungen der Formel III in eine Verbindung der Formel I kann in unterschiedlicher Reihenfolge vorgenommen werden:
   1) Veresterung der Alkoholfunktion (R¹ = Wasserstoff) → Modifikation von R⁴ und/oder R⁵ → gegenenfalls Epoxidöffnung, falls R² und R³ gemeinsam ein Sauerstoffatom darstellen, gegebenfalls mit anschließender Modifikation von R² und R³.
   2) Veresterung der Alkoholfunktion (R¹ = Wasserstoff) → gegenenfalls Epoxidöffnung, falls R² und R³ gemeinsam ein Sauerstoffatom darstellen, gegebenfalls mit anschließender Modifikation von R² und R³ → Modifikation von R⁴ und/oder R⁵.
   3) Schutz der Alkoholfunktion (R¹ = Wasserstoff) → gegenenfalls Epoxidöffnung, falls R² und R³ gemeinsam ein Sauerstoffatom darstellen, gegebenfalls mit anschließender Modifikation von R² und R³ → Modifikation von R⁴ und/oder R⁵ → Freisetzung und anschließende Veresterung der Alkoholfunktion (R¹ = Wasserstoff).
   4) Schutz der Alkoholfunktion (R¹ = Wasserstoff) → Modifikation von R⁴ und/oder R⁵ → Freisetzung und anschließende Veresterung der Alkoholfunktion (R¹ = Wasserstoff) → gegenenfalls Epoxidöffnung, falls R² und R³ gemeinsam ein Sauerstoffatom darstellen, gegebenfalls mit anschließender Modifikation von R² und R³.

Zur Veresterung der Alkoholfünktion (R¹ = Wasserstoff) wird mit einer Base wie z.B. Metallhydriden (z.B. Natriumhydrid), Alkalialkoholaten (z.B. Natriummethanolat, Kalium-tert.-butanolat), Alkalihexamethyldisilazan (z.B. Natriumhexamethyldisilazan), 1,5-Diazabicyclo[4.3.0]non-5-en (DBN), 1,8-Diazabicyclo[5.4.0]undec-7-en (DBU), Triethylamin, 4-(Dimethylamino)pyridin (DMAP), 1,4-Diazabicyclo[2.2.2]octan (DABCO) deprotoniert und mit geeigneten Carbonsäurederivaten wie z.B. Säureamiden, Säurehalogeniden, Säureanhydriden in einem inerten Solvens wie z.B. Dichlormethan, Diethylether, Tetrahydrofuran bei -70°C bis +50°C umgesetzt. Bevorzugt wird die Umsetzung mit Natriumhexamethyldisilazan als Base, einem cyclischen Säureamid als Carbonsäurederivat, Tetrahydrofuran als Solvens bei Temperaturen von -40°C bis +25°C.
Falls R⁴ und R⁵ gemeinsam eine =CHR¹⁰-Gruppe darstellen, kann die Funktionalisierung der olefinischen Doppelbindung nach den, dem Fachmann bekannten Methoden erfolgen. Beispielsweise kann Wasserstoff angelagert werden z.B. durch kat. Hydrierung, es können Hydroxylgruppen eingeführt werden durch Wasseranlagerung (Hydroborierung, Oxymercurierung) oder durch 1.2-Bishydroxylierung z.B. mit Osmiumtetroxid oder Kaliumpermanganat. Die Einführung einer Carbonylgruppe (R⁴, R⁵ stellen gemeinsam ein Sauerstoffatom dar) gelingt nach Spaltung der Doppelbindung z.B. durch Ozonolyse oder durch oxidative Spaltung eines 1.2-Diols. Eine derartig erzeugte Carbonylgruppe kann beispielsweise reduziert, alkyliert oder als Carbonylkomponente in einer Wittig-Reaktion zum Aufbau modifizierter =CHR¹⁰-Gruppen verwendet werden.
Falls R² und R³ gemeinsam ein Sauerstoffatom darstellen, kann das Epoxid durch Nucleophile wie beispielsweise Wasser, Carbonsäurederivate (Carbonsäuren, Carbonsäurehalogenide, Carbonsäureanhydride), Sulfonsäurederivate (Sulfonsäuren, Sulfonsäurehalogenide, Sulfonsäureanhydride), Amine, in Gegenwart von mineralischen oder organischen Säuren wie beispielsweise Chlorwasserstoff, para-Toluolsulfonsäure oder Lewissäuren wie beispielsweise Bortrifluoridetherat, Titantetraisopropoxid, Cerammoniumnitrat entweder in inerten oder als Nucleophil fungierenden Lösungsmitteln bei -70°C bis +50°C umgesetzt werden.

### Biologische Wirkungen und Anwendungsbereiche der neuen Borneolderivate:

Die neuen Verbindungen der Formel I sind wertvolle Pharmaka. Sie interagieren mit Tubulin, indem sie gebildete Mikrotubuli stabilisieren und sind somit in der Lage, die Zellteilung phasenspezifisch zu beeinflussen. Dies betrifft vor allem schnell wachsende, neoplastische Zellen, deren Wachstum durch interzelluläre Regelmechnismen weitgehend unbeeinflußt ist. Wirkstoffe dieser Art sind prinzipiell geeignet zur Behandlung maligner Tumoren. Als Anwendungsbereich seien beispielweise genannt die Therapie von Ovarial-, Magen-, Colon-, Adeno-, Brust-, Lungen-, Kopf- und Nacken-Karzinomen, dem malignen Melanom, der akuten lymphozytären und myelocytären Leukämie. Die erfindungsgemäßen Verbindungen können alleine oder zur Erzielung additiver oder synergistischer Wirkungen in Kombination mit weiteren in der Tumortherapie anwendbaren Prinzipien und Substanzklassen verwendet werden.
Als Beispiele seien genannt die Kombination mit
○ Platinkomplexen wie z.B. Cisplatin, Carboplatin,
○ interkalierenden Substanzen z.B. aus der Klasse der Anthracycline wie z.B. Doxorubicin oder aus der Klasse der Antrapyrazole wie z.B. CI-941,
○ mit Tubulin interagierenden Substanzen z.B. aus der Klasse der Vinka-Alkaloide wie z.B. Vincristin, Vinblastin oder aus der Klasse der Taxane wie z.B. Taxol, Taxotere oder aus der Klasse der Makrolide wie z.B. Rhizoxin oder andere Verbindungen wie z.B. Colchicin, Combretastatin A-4,
○ DNA Topoisomeraseinhibitoren wie z.B. Camptothecin, Etoposid, Topotecan, Teniposid,
○ Folat- oder Pyrimidin-Antimetaboliten wie z.B. Lometrexol, Gemcitubin,
○ DNA alkylierenden Verbindungen wie z.B. Adozelesin, Dystamycin A,
○ Inhibitoren von Wachstumsfaktoren (z.B. von PDGF, EGF, TGFβ, EGF) wie z.B. Somatostatin, Suramin, Bombesin-Antagonisten,
○ Inhibitoren der Protein Tyrosin Kinase oder der Protein Kinasen A oder C wie z.B. Erbstatin, Genistein, Staurosporin, Ilmofosin, 8-Cl-cAMP,
○ Antihormonen aus der Klasse der Antigestagene wie z.B. Mifepriston, Onapriston oder aus der Klasse der Antiöstrogene wie z.B. Tamoxifen oder aus der Klasse der Antiandrogene wie z.B. Cyproteronacetat,
○ Metastasen inhibierenden Verbindungen z.B. aus der Klasse der Eicosanoide wie z.B. PGI₂, PGE₁, 6-Oxo-PGE₁ sowie deren stabiler Derivate (z.B. Iloprost, Cicaprost).

Die Erfindung betrifft auch Arzneimittel auf Basis der pharmazeutisch verträglichen, d.h. in den verwendeten Dosen nicht toxischen Verbindungen der allgemeinen Formel I, gegebenenfalls zusammen mit den üblichen Hilfs- und Trägerstoffen.

Die erfindungsgemäßen Verbindungen können nach an sich bekannten Methoden der Galenik zu pharmazeutischen Präparaten für die enterale, percutane, parenterale oder lokale Applikation verarbeitet werden. Sie können in Form von Tabletten, Dragees, Gelkapscln, Granulaten, Suppositorien, implantaten, injizierbaren sterilen wäßrigen oder öligen Lösungen, Suspensionen oder Emulsionen, Salben, Cremes und Gelen verabreicht werden.
Der oder die Wirkstoffe können dabei mit den in der Galenik üblichen Hilfsstoffen wie z.B. Gummiarabikum, Talk, Stärke, Mannit, Methylcellulose, Laktose, Tensiden wie Tweens oder Myrj, Magnesiumstearat, wäßrigen oder nicht wäßrigen Trägern, Paraffinderivaten, Netz-, Dispergier-, Emulgier-, Konservierungsmitteln und Aromastoffen zur Geschmackskorrektur (z.B. etherischen Ölen) gemischt werden.
Die Erfindung betrifft somit auch pharmazeutische Zusammensetzungen, die als Wirkstoff zumindest eine erfindungsgemäße Verbindung enthalten. Eine Dosiseinheit enthält etwa 0,1-100 mg Wirkstoff(e). Die Dosierung der erfindungsgemäßen Verbindungen liegt beim Menschen bei etwa 0,1-1000 mg pro Tag.

Die nachfolgenden Ausführungsbeispiele dienen zur weiteren Erläuterung des erfindungsgemäßen Verfahrens.

### BEISPIEL 1

### [1R-[1α,2β(2R*,3S*),4α,4aβ,10aβ]]-3-[[(1,1-Dimethylethoxy)carbonyl]amino]-2-hydroxybenzenpropansäure-9-methylen-1,2,3,4,4a,9,10,10a-octahydro-1,12,12-trimethyl-4a,10a-epoxy-1,4-methanophenanthren-2-ylester

150 mg (214 µmol) der nach Beispiel la dargestellten polaren Verbindung A löst man unter einer Atmosphäre aus trockenem Argon in 7 ml wasserfreiem Tetrahydrofuran, versetzt mit 0,29 ml einer 1,1 M Lösung von Tetrabutylammoniumfluorid in Tetrahydrofuran und rührt 1 Stunde bei 23°C. Man engt ein und reinigt den Rückstand durch Chromatographie an ca. 40 ml feinem Kieselgel mit einem Gradientensystem aus n-Hexan und Ethylacetat. Isoliert werden 113 mg (207 µmol, 97%) der Titelverbindung als farbloser Schaum.
¹H-NMR (DMSO-d6, 80°C): δ = 0,78 (3H), 0,91 (3H), 1,02 (3H), 1,32 (9H), 1,58 (1H), 2,17 (1H), 2,75 (2H), 3,08 (1H), 4,36 (1H), 4,93 (1H), 5,01 (1H), 5,07 (1H), 5,33 (1H), 5,49 (1H), 6,53 (1H), 7,20-7,38 (7H), 7,56 (2H) ppm.

### BEISPIEL 1a

### [1R-[1α,2β(2R*,3S*),4α,4aβ,10aβ]]-3-[[(1,1-Dimethylethoxy)carbonyl]amino]-2-(triisopropyisilyloxy)benzenpropansäure-9-methylen-1,2,3,4,4a,9,10,10a-octahydro-1,12,12-trimethyl-4a,10a-epoxy-1,4-methanophenanthren-2-ylester (A) und [1S-[1α,2β(2R*,3S*),4α,4aβ,10aβ]]-3-[[(1,1-Dimethylethoxy)carbonyl]amino]-2-(trüsopropylsilyloxy)benzenpropansäure-9-methylen-1,2,3,4,4a,9,10,10a-octahydro-1,12,12-trimethyl-4a,10a-epoxy-1,4-methanophenanthren-2-ylester (B)

79 mg (280 µmol) des nach Beispiel 1b dargestellten Gemisches sowie 176 mg des nach Beispiel 1c dargestellten β-Lactams löst man unter einer Atmosphäre aus trockenem Argon in 30 ml wasserfreiem Tetrahydrofuran, versetzt bei -35°C mit 0,34 ml einer 1M Lösung von Natriumhexamethyldisilazan in Tetrahydrofuran und rührt 15 Minuten nach. Man gießt in gesättigte Ammoniumchloridlösung, extrahiert mehrfach mit Ethylacetat, wäscht die vereinigten organischen Extrakte mit Wasser und gesättigter Natriumchloridlösung und trocknet über Natriumsulfat, Den nach Filtration und Lösungsmittelabzug erhaltenen Rückstand trennt man durch Chromatographie an ca. 70 ml feinem Kieselgel mit einem Gradientensystem aus n-Hexan und Ethylacetat. Isoliert werden 26 mg (37 µmol, 13%) der Titelverbindung B als unpolare Komponente sowie 91 mg (130 µmol, 46%) der Titelverbindung A als polare Komponente, jeweils als farbloser Schaum.
¹H-NMR (CDCl₃) von A: δ = 0,8-1,47 (39H), 1,71 (1H), 2,22 (1H), 2,64 (1H), 2,71 (1H), 3,03 (1H), 4,66 (1H), 4,96 (1H), 5,02 (1H), 5,16 (1H), 5,47 (1H), 5,92 (1H), 7,16-7,54 (9H) ppm.
¹H-NMR (CDCl₃) von B: δ = 0,8-1,47 (39H), 1,79 (1H), 2,38 (1H), 2,68 (1H), 2,70 (1H), 3,15 (1H), 4,69 (1H), 5,06 (1H), 5,08 (1H), 5,22 (1H), 5,50 (1H), 5,71 (1H), 7,18-7,56 (9H) ppm.

### BEISPIEL 1b

### [1R-(1α,2β,4α,4aβ,10aβ)]-9-Methylen-1,2,3,4,4a,9,10,10a-octahydro-1,12,12-trimethyl-4a,10a-epoxy-1,4-methanophenanthren-2-ol (A) und [1S-(1α,2β,4α,4aβ, 10aβ)]-9-Methylen-1,2,3,4,4a,9,10,10a-octahydro-1,12,12-trimethyl-4a,10a-epoxy-1,4-methanophenanthren-2-ol (B)

2,1g (7,88 mmol) eines ca. 7:3-Gemisches aus [4R-(4α,4aβ,10aα)]-9-Methylen-3,4,4a,9,10,10a-hexahydro-1,11,11-trimethyl-4,10a-methanophenanthren-4a-ol und [4S-(4α,4aβ,10aα)]-9-Methylen-3,4,4a,9,10,10a-hexahydro-1,11,11-trimethyl-4,10a-methanophenanthren-4a-ol, das man in Analogie zu dem im J. Am. Chem. Soc. 1992, auf Seite 5879 beschriebenen Verfahren hergestellt hat, löst man unter einer Atmosphäre aus trockenem Argon in 80 ml wasserfreiem Dichlormethan und kühlt auf 0°C. Man versetzt mit 2,4 ml Titan(IV)isopropylat, 1,5 ml einer 6,5 M wasserfreien Lösung von tert.-Butylhydroperoxid in Toluol und rührt 0,5 Stunden. Man gießt in Wasser, extrahiert mehrfach mit Diethylether, wäscht die vereinigten organischen Extrakte mit Wasser und gesättigter Natriumchloridlösung und trocknet über Natriumsulfat. Den nach Filtration und Lösungsmittelabzug erhaltenen Rückstand trennt man durch Chromatographie an ca. 200 ml feinem Kieselgel mit einem Gradientensystem aus n-Hexan und Ethylacetat. Isoliert werden 1,39 g (4,92 mmol, 62%) der Titelverbindungen A und B als kristalliner Feststoff. Durch Kristallisation aus Diisopropylether läßt sich das im Überschuß in dem Produktgemisch enthaltene Isomer A enantiomerenrein gewinnen.
¹H-NMR (CDCl₃): δ = 0,79 (3H), 0,88 (3H), 1,11 (3H), 1,58 (1H), 2,38 (1H), 2,68 (1H), 2,74 (1H), 3,08 (1H), 3,79 (1H), 3,97 (1H), 5,02 (1H), 5,48 (1H), 7,30 (2H), 7,51 (2H) ppm.

### BEISPIEL 1c

### (3R,4S)-1-[(1,1-dimethylethoxy)carbonyl]-3-triisopropylsilyloxy-4-phenyl-2-azetidinon

2,5 g (7,82 mmol) (3R,4S)-3-Triisopropylsilyloxy-4-phenylazetidin-2-on, das man in Analogie zu dem in Tetrahedron 48, 6985 (1992) beschriebenen Verfahren hergestellt hat, löst man unter einer Atmosphäre aus trockenem Argon in 50 ml wasserfreiem Dichlormethan, kühlt auf 0°C, versetzt mit 3,1 ml Triethylamin, 3,65 ml Pyrokohlensäure-di-tert.butylether sowie einer katalytischen Menge Dimethylaminopyridin. Man läßt auf 23°C erwärmen und 16 Stunden rühren. Man gießt in gesättigte Ammoniumchloridlösung, extrahiert mehrfach mit Diethylether, wäscht die vereinigten organischen Extrakte mit Wasser und gesättigter Natriumchloridlösung und trocknet über Natriumsulfat. Den nach Filtration und Lösungsmittelabzug erhaltenen Rückstand trennt man durch Chromatographie an ca. 300 ml feinem Kieselgel mit einem Gradientensystem aus n-Hexan und Ethylacetat. Isoliert werden 3,1 g (7,39 mmol, 94%) der Titelverbindung als kristalliner Feststoff.
¹H-NMR (CDCl₃): δ = 0,80-1,02 (21H), 1,40 (9H), 5,07 (1H), 5,17 (1H), 7,27-7,40 (5H) ppm.

### BEISPIEL 1d

### (3R,4S)-1-benzoyl-3-triisopropylsilyloxy-4-phenyl-2-azetidinon

5,0 g (15,6 mmol) (3R,4S)-3-Triisopropylsilyloxy-4-phenylazetidin-2-on, das man in Analogie zu dem in Tetrahedron 48, 6985 (1992) beschriebenen Verfahren hergestellt hat, löst man unter einer Atmosphäre aus trockenem Argon in 100 ml wasserfreiem Dichlormethan, kühlt auf 0°C, versetzt mit 6,2 ml Triethylamin, 3,85 ml Benzoylchlorid sowie einer katalytischen Menge dimethylaminopyridin. Man läßt auf 23°C erwärmen und 16 Stunden rühren. Man gießt in gesättigte Ammoniumchloridlösung, extrahiert mehrfach mit Diethylether, wäscht die vereinigten organischen Extrakte mit Wasser und gesättigter Natriumchloridlösung und trocknet über Natriumsulfat. Den nach Filtration und Lösungsmittelabzug erhaltenen Rückstand trennt man durch Chromatographie an ca. 400 ml feinem Kieselgel mit einem Gradientensystem aus n-Hexan und Ethylacetat. Isoliert werden 6,43 g (15,2 mmol, 97%) der Titelverbindung als kristalliner Feststoff. ¹H-NMR (CDCl₃): δ = 0,80-1,07 (21H), 5,25 (1H), 5,43 (1H), 7,27-7,43 (5H), 7,48 (2H), 7,59 (1H), 8,03 (2H) ppm.

### BEISPIEL 1e

### (3R,4S)-1-Cyclohexylcarbonyl-3-triisopropylsilyloxy-4-phenyl-2-azetidinon

2,0 g (6,3 mmol) (3R,4S)-3-Triisopropylsilyloxy-4-phenylazetidin-2-on, das man in Analogie zu dem in Tetrahedron 48, 6985 (1992) beschriebenen Verfahren hergestellt hat, setzt man in Analogie zu Beispiel 1d unter Verwendung von Cyclohexancarbonsäurechlorid um und isoliert nach Aufarbeitung und Reinigung 2,6 g (6,1 mmol, 96%) der Titelverbindung.
¹H-NMR (CDCl₃): δ = 0,81-1,03 (21H), 1,12-2,04 (10H), 3,03 (1H), 5,14 (1H), 5,19 (1H), 7,18-7,37 (5H) ppm.

### BEISPIEL 1f

### (3R,4S)-1-Acetyl-3-triisopropylsilyloxy-4-phenyl-2-azetidinon

2,0 g (6,3 mmol) (3R,4S)-3-Triisopropylsilyloxy-4-phenylazetidin-2-on, das man in Analogie zu dem in Tetrahedron 48, 6985 (1992) beschriebenen Verfahren hergestellt hat, setzt man in Analogie zu Beispiel 1d unter Verwendung von Acetylchlorid um und isoliert nach Aufarbeitung und Reinigung 1,8 g (5,0 mmol, 79%) der Titelverbindung.
¹H-NMR (CDCl₃): δ = 0,77-1,04 (21H), 2,45 (3H), 5,17 (1H), 5,22 (1H), 7,20-7,39 (5H) ppm.

### BEISPIEL 2

### [1S-[1α,2β(2R*,3S*),4α,4aβ,10aβ]]-3-[[(1,1-Dimethylethoxy)carbonyl]amino]-2-hydroxybenzenpropansäure-9-methylen-1,2'3,4,4a'9,10,10a-octahydro-1,12,12-trimethyl-4a,10a-epoxy-1,4-methanophenanthren-2-ylester

In Analogie zu Beispiel 1 werden 26 mg (37 µmol) der nach Beispiel la dargestellten unpolaren Verbindung B umgesetzt. Nach Aufarbeitung und Reinigung isoliert man 14 mg (25 µmol, 69%) der Titelverbindung als farblosen Schaum.
¹H-NMR (CDCl₃): δ = 0,84 (3H), 0,96 (3H), 1,08 (3H), 1,42 (9H), 1,83 (1H), 2,40 (1H), 2,70 (1H), 2,72 (1H), 3,18 (1H), 3,21 (1H), 4,58 (1H), 5,02 (1H), 5,10 (1H), 5,28 (1H), 5,50 (1H), 5,55 (1H), 7,22-7,38 (9H) ppm.

### BEISPIEL 3

### [1R-[1α,2β(2R*,3S*),4α,4aβ,10aβ]]-3-[benzoylamino]-2-hydroxybenzenpropansäure-9-methylen-1,2,3,4,4a,9,10,10a-octahydro-1,12,12-trimethyl-4a,10a-epoxy-1,4-methanophenanthren-2-ylester

100 mg (142 µmol) der nach Beispiel 3a dargestellten polaren Verbindung A setzt man in Analogie zu Beispiel 1 um und isoliert nach Aufarbeitung und Reinigung 66 mg (120 µmol, 85%) der Titelverbindung als farblosen Schaum.
¹H-NMR (CDCl₃): δ = 0,82 (3H), 0,91 (3H), 0,98 (3H), 1,81 (1H), 2,29 (1H), 2,68 (1H), 2,71 (1H), 2,91 (1H), 3,59 (1H), 4,71 (1H), 4,88 (1H), 5,13 (1H), 5,40 (1H), 5,71 (1H), 7,18 (2H), 7,22-7,40 (6H), 7,40-7,59 (4H), 7,70 (1H), 7,87 (2H) ppm.

### BEISPIEL 3a

### [1R-[1α,2β(2R*,3S*),4α,4aβ,10aβ]]-3-[benzoylamino]-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester (A) und [1S-[1α,2β(2R*,3S*), 4α,4aβ,10aβ]]-3-[benzoylamino]-2-(triisopropylsilyloxy) benzenpropansäure-9-methylen-1,2,3,4,4a,9,10,10a-octahydro-1,12,12-trimethyl-4a,10a-epoxy-1,4-methanophenanthren-2-ylester (B)

80 mg (283 µmol) des nach Beispiel 1b dargestellten Gemisches sowie 181 mg des nach Beispiel 1d dargestellten β-Lactams setzt man in Analogie zu Beispiel 1a um und isoliert nach Aufarbeitung und Reinigung 24 mg (34 µmol, 12%) der Titelverbindung B als unpolare Komponente sowie 100 mg (142 µmol, 50%) der Titelverbindung A als polare Komponente, jeweils als farblosen Schaum.
¹H-NMR (CDCl₃) von A: δ = 0,8-1,22 (30H), 1,72 (1H), 2,20 (1H), 2,64 (1H), 2,66 (1H), 2,95 (1H), 4,78 (1H), 4,92 (1H), 5,05 (1H), 5,42 (1H), 5,68 (1H), 7,17-7,58 (12H), 7,65 (1H), 7,92 (2H) ppm.
¹H-NMR (CDCl₃) von B: δ = 0,77 (3H), 0,80 (3H), 0,85-1,18 (24H), 1,65 (1H), 2,28 (1H), 2,53 (1H), 2,61 (1H), 2,79 (1H), 4,78 (1H), 5,02 (1H), 5,15 (1H), 5,46 (1H), 5,78 (1H), 7,11 (2H), 7,21-7,57 (10H), 7,74 (1H), 7,92 (2H) ppm.

### BEISPIEL 4

### [1S-[1α,2β(2R*,3S*),4α,4aβ,10aβ]]-3-[benzoylamino]-2-hydroxybenzenpropansäure-9-methylen-1,2,3,4,4a,9,10,10a-octahydro-1,12,12-trimethyl-4a,10a-epoxy-1,4-methanophenanthren-2-ylester

24 mg (34 µmol) der nach Beispiel 3a dargestellten unpolaren Verbindung B setzt man in Analogie zu Beispiel 1 um und isoliert nach Aufarbeitung und Reinigung 10 mg (18 µmol, 54%) der Titelverbindung als farblosen Schaum.
¹H-NMR (CDCl₃): δ = 0,81 (3H), 0,90 (6H), 1,76 (1H), 2,32 (1H), 2,63 (1H), 2,67 (1H), 3,00 (1H), 3,34 (1H), 4,69 (1H), 5,07 (1H), 5,12 (1H), 5,47 (1H), 5,90 (1H), 7,16-7,58 (13H), 7,87 (2H) ppm.

### BEISPIEL 5

### [1S-[1α,2β(2R*,3S*),4α,4aβ,10aβ]]-3-[[(1,1-Dimethylethoxy)carbonyl]amino]-2-hydroxybenzenpropansäure-9-methylen-1,2,3,4,4a,9,10,10a-octahydro-1,11,11-trimethyl-4a-methoxy-10a-hydroxy-1,4-methanophenanthren-2-ylester

22 mg (30 µmol) der nach Beispiel 5a dargestellten Verbindung setzt man in Analogie zu Beispiel 1 um und isoliert nach Aufarbeitung und Reinigung 9 mg (16 µmol, 52%) der Titelverbindung als farblosen Schaum.
¹H-NMR (CDCl₃): δ = 0,65 (3H), 1,01 (6H), 1,43 (9H), 2,07 (1H), 2,41 (1H), 2,57 (1H), 2,73 (2H), 2,82 (3H), 3,27 (1H), 3,82 (1H), 4,51 (1H), 5,12 (1H), 5,21 (2H), 5,33 (1H), 6,23 (1H), 7,22-7,50 (9H) ppm.

### BEISPIEL 5a

### [1S-[1α,2β(2R*,3S*),4α,4aβ,10aβ]]-3-[[(1,1-Dimethylethoxy)carbonyl]amino]-2-(triisopropylsilyloxy)benzenpropansäure-9-methylen-1,2,3,4,4a,9,10,10a-octahydro-1,11,11-trimethyl-4a-methoxy-10a-hydroxy-1,4-methanophenanthren-2-ylester

20 mg (28 µmol) der nach Beispiel la dargestellten unpolaren Verbindung B löst man in einem Gemisch aus 0,2 ml Dichlormethan und 2 ml Methanol, versetzt mit 200 mg DOWEX 50 WX 4 und rührt unter einer Atmosphäre aus trockenem Argon 5 Stunden bei 23°C. Man filtriert, wäscht das Filtrat mit gesättigter Natriumhydrogencarbonatlösung, gesättigter Natriumchloridlösung und trocknet über Magnesiumsulfat. Nach Fütration und Lösungsmittelabzug isoliert man 12 mg (16 µmol, 57%) der Titelverbindung als farblosen Schaum, den man ohne Reinigung weiter umsetzt.
¹H-NMR (CDCl₃): δ = 0,64 (3H), 0,80-1,09 (27H), 1,44 (9H), 2,09 (1H), 2,43 (1H), 2,57 (1H), 2,68 (1H), 2,75 (1H), 2,80 (3H), 3,70 (1H), 4,60 (1H), 5,10 (1H), 5,12 (1H), 5,20 (1H), 5,34 (1H), 6,08 (1H), 7,17-7,49 (9H) ppm.

### BEISPIEL 6

### [1R-[1α,2β(2R*,3S*),4α,4aβ,10aβ]]-3-[[(1,1-Dimethylethoxy)carbonyl]amino]-2-hydroxybenzenpropansäure-9-methylen-1,2,3,4,4a,9,10,10a-octahydro-1,11,11-trimethyl-4a-methoxy-10a-hydroxy-1,4-methanophenanthren-2-ylester

15,6 mg (21 µmol) der nach Beispiel 6a dargestellten Verbindung setzt man in Analogie zu Beispiel 1 um und isoliert nach Aufarbeitung und Reinigung 8,6 mg (15 µmol, 71%) der Titelverbindung als farblosen Schaum.
¹H-NMR (CDCl₃): δ = 0,65 (3H), 1,01 (6H), 1,43 (9H), 2,17 (1H), 2,38 (1H), 2,58 (1H), 2,73 (2H), 2,86 (3H), 3,11 (1H), 3,92 (1H), 4,56 (1H), 5,11 (1H), 5,21 (2H), 5,35 (1H), 6,21 (1H), 7,20-7,50 (9H) ppm.

### BEISPIEL 6a

### [1R-[1α,2β(2R*,3S*),4α,4aβ,10aβ]]-3-[[(1,1-Dimethylethoxy)carbonyl]amino]-2-(triisopropylsilyloxy)benzenpropansaure-9-methylen-1,2,3,4,4a,9,10,10a-octahydro-1,11,11-trimethyl-4a-methoxy-10a-hydroxy-1,4-methanophenanthren-2-ylester

15 mg (21 µmol) der nach Beispiel la dargestellten polaren Verbindung A setzt man in Analogie zu Beispiel 5a um und isoliert nach Aufarbeitung 15,7 mg (21 µmol, 100%) der Titelverbindung als farblosen Schaum, der ohne Reinigung weiter umgesetzt wird.
¹H-NMR (CDCl₃): δ = 0,63 (3H), 0,78-1,04 (27H), 1,43 (9H), 2,03 (1H), 2,30 (1H), 2,58 (1H), 2,68 (1H), 2,74 (1H), 2,80 (3H), 3,82 (1H), 4,69 (1H), 5,11 (1H), 5,14 (1H), 5,27 (1H), 5,34 (1H), 6,39 (1H), 7,16-7,48 (9H) ppm.

### BEISPIEL 7

### [1R-[1α,2β(2R*,3S*),4α,4aβ,10aβ]]-3-[[(1,1-Dimethylethoxy)carbonyl]amino]-2-hydroxybenzenpropansäure-9-methylen-1,2,3,4,4a,9,10,10a-octahydro-1,11,11-trimethyl-4a-ethoxy-10a-hydroxy-1,4-methanophenanthren-2-ylester

16 mg (21 µmol) der nach Beispiel 7a dargestellten Verbindung setzt man in Analogie zu Beispiel 1 um und isoliert nach Aufarbeitung und Reinigung 7,8 mg (13 µmol, 63%) der Titelverbindung als farblosen Schaum.
¹H-NMR (CDCl₃): δ = 0,64 (3H), 1,01 (6H), 1,06 (3H), 1,41 (9H), 2,22-2,43 (2H), 2,58 (1H), 2,68-2,90 (3H), 2,93-3,13 (2H), 4,04 (1H), 4,56 (1H), 5,12 (1H), 5,18 (1H), 5,32 (2H), 6,36 (1H), 7,18-7,50 (9H) ppm.

### BEISPIEL 7a

### [1R-[1α,2β(2R*,3S*),4α,4aβ,10aβ]]-3-[[(1,1-Dimethylethoxy)carbonyl]amino]-2-(triisopropylsilyloxy)benzenpropansäure-9-methylen-1,2,3,4,4a,9,10,10a-octahydro-1,11,11-trimethyl-4a-ethoxy-10a-hydroxy-1,4-methanophenanthren-2-ylester

15 mg (21 µmol) der nach Beispiel la dargestellten polaren Verbindung A setzt man in Analogie zu Beispiel 5a unter Verwendung von Ethanol um und isoliert nach Aufarbeitung 16 mg (21 µmol, 100%) der Titelverbindung, die man ohne Reinigung weiter umsetzt, als farblosen Schaum.
¹H-NMR (CDCl₃): δ = 0,63 (3H), 0,78-1,12 (30H), 1,43 (9H), 2,13-2,37 (2H), 2,57 (1H), 2,64-2,86 (3H), 3,08 (1H), 3,98 (1H), 4,69 (1H), 5,12 (2H), 5,33 (2H), 6,33 (1H), 7,15-7,48 (9H) ppm.

### BEISPIEL 8

### [1S-[1α,2β(2R*,3S*),4α,4aβ,10aβ]]-3-[[(1,1-Dimethylethoxy)carbonyl]aminol-2-hydroxybenzenpropansäure-9-methylen-1,2,3,4,4a,9,10,10a-octahydro-1,11,11-trimethyl-4a,10a-dihydroxy-1,4-methanophenanthren-2-ylester

6,5 mg (9,0 µmol) der nach Beispiel 8a dargestellten Verbindung setzt man in Analogie zu Beispiel 1 um und isoliert nach Aufarbeitung und Reinigung 3,5 mg (6,2 µmol, 69%) der Titelverbindung als farblosen Schaum.
¹H-NMR (CDCl₃): δ = 0,69 (3H), 1,01 (3H), 1,07 (3H), 1,48 (9H), 2,26-2,49 (2H), 2,54 (1H), 2,76 (1H), 2,88 (1H), 3,25 (1H), 4,40 (1H), 4,54 (1H), 4,84 (1H), 5,08 (1H), 5,34 (1H), 5,37 (1H), 5,53 (1H), 5,65 (1H), 7,20-7,49 (9H) ppm.

### BEISPIEL 8a

### [1S-[1α,2β(2R*,3S*),4α,4aβ,10aβ]]-3-[[(1,1-Dimethylethoxy)carbonyl]amino]-2-(triisopropylsilyloxy)benzenpropansäure-9-methylen-1,2,3,4,4a,9,10,10a-octahydro-1,11,11-trimethyl-4a,10a-dihydroxy-1,4-methanophenanthren-2-ylester

14 mg (20 µmol) der nach Beispiel la dargestellten unpolaren Verbindung B löst man in 1,5 ml Tetrahydrofuran, versetzt mit 0,5 ml Wasser, 3 Tropfen einer 4N Salzsäure und rührt unter einer Atmosphäre aus Argon 16 Stunden bei 23°C. Man gießt in gesättigte Natriumhydrogencarbonatlösung, extrahiert mit Diethylether, wäscht mit gesättigter Natriumchloridlösung und trocknet über Magnesiumsulfat Den nach Filtration und Lösungsmittelabzug erhaltenen Rücklstand reinigt man durch Chromatographie an einer analytischen Dünnschichtplatte. Als Laufmittel dient ein Gemisch aus n-Hexan und Ethylacetat, als Elutionsmittel Diethylether. Isoliert werden 4,2 mg (5,8 µmol, 29%) der Titelverbindung als farbloser Schaum sowie 8,6 mg Ausgangsmaterial.
¹H-NMR (CDCl₃): δ = 0,70 (3H), 0,80-1,08 (27H), 1,45 (9H), 2,31 (1H), 2,49 (1H), 2,54 (1H), 2,72 (1H), 2,86 (1H), 3,99 (1H), 4,54 (1H), 4,63 (1H), 5,08 (1H), 5,22-5,40 (3H), 5,75 (1H), 7,18-7,47 (9H) ppm.

### BEISPIEL 9

### [1R-[1α,2β(2R*,3S*),4α,4aβ,10aβ]]-3-[[(1,1-Dimethylethoxy)carbonyl]amino]-2-hydroxybenzenpropansäure-9-methylen-1,2,3,4,4a,9,10,10a-octahydro-1,11,11-trimethyl-4a,10a-dihydroxy-1,4-methanophenanthren-2-ylester

6,0 mg (8,3 µmol) der nach Beispiel 9a dargestellten Verbindung setzt man in Analogie zu Beispiel 1 um und isoliert nach Aufarbeitung und Reinigung 3,0 (5,3 µmol, 64%) der Titelverbindung als farblosen Schaum.
¹H-NMR (CDCl₃): δ = 0,71 (3H), 1,04 (6H), 1,40 (9H), 2,22-2,48 (2H), 2,57 (1H), 2,73 (1H), 2,89 (1H), 3,01 (1H), 3,12 (1H), 3,95 (1H), 4,54 (1H), 5,12 (1H), 5,21 (2H), 5,37 (1H), 5,93 (1H), 7,22-7,50 (9H) ppm.

### BEISPIEL 9a

### [1R-[1α,2β(2R*,3S*),4α,4aβ,10aβ]]-3-[[(1,1-Dimethylethoxy)carbonyl]amino]-2-(triisopropylsilyloxy)benzenpropansäure-9-methylen-1,2,3,4,4a,9,10,10a-octahydro-1,11,11-trimethyl-4a,10a-dihydroxy-1,4-methanophenanthren-2-ylester

15 mg (21,4 µmol) der nach Beispiel la dargestellten polaren Verbindung A setzt man in Analogie zu Beispiel 8a um und isoliert nach Aufarbeitung und Reinigung 6 mg (8,3 µmol,39%) der Titelverbindung als farblosen Schaum.
¹H-NMR (CDCl₃): δ = 0,70 (3H), 0,78-1,50 (36H), 2,18-2,40 (2H), 2,56 (1H), 2,71 (1H), 2,86 (1H), 3,26 (1H), 4,25 (1H), 4,68 (1H), 5,12 (2H), 5,34 (2H), 5,99 (1H), 7,16-7,47 (9H) ppm.

### BEISPIEL 10

### [1R-[1α,2β(2R*,3S*),4α,4aβ,10aβ]]-3-[[Cyclohexylcarbonyl]amino]-2-hydroxybenzenpropansäure-9-methylen-1,2,3,4,4a,9,10,10a-octahydro-1,12,12-trimethyl-4a,10a-epoxy-1,4-methanophenanthren-2-ylester

38 mg (53 µmol) der nach Beispiel 10a dargestellten Verbindung setzt man in Analogie zu Beispiel 1 um und isoliert nach Aufarbeitung und Reinigung 24 mg (43 µmol, 81%) der Titelverbindung als farblosen Schaum.
¹H-NMR (CDCl₃): δ = 0,60 (1H), 0,87 (3H), 0,93 (3H), 0,97-1,85 (14H), 2,22 (1H), 2,35 (1H), 2,76 (1H), 2,82 (1H), 3,10 (1H), 4,53 (1H), 5,08 (1H), 5,10 (1H), 5,47 (1H), 5,54 (1H), 7,19-7,45 (8H), 7,52 (1H), 7,58 (1H) ppm.

### BEISPIEL 10a

### [1R-[1α,2β(2R*,3S*),4α,4aβ,10aβ]]-3-[[Cyclohexylcarbonyl]amino]-2-(triisopropylsilyloxy)benzenpropansäure-9-methylen-1,2,3,4,4a,9,10,10a-octahydro-1,12,12-trimethyl-4a,10a-epoxy-1,4-methanophenanthren-2-ylester

15 mg (53 µmol) der nach Beispiel 1b dargestellten und durch Kristallisation enantiomerenrein erhaltenen Verbindung A sowie 34 mg des nach Beispiel le dargestellten β-Lactams setzt man in Analogie zu Beispiel 1a um und isoliert nach Aufarbeitung 44 mg der Titelverbindung als Rohprodukt, das man ohne Reinigung weiter umsetzt.
¹H-NMR (CDCl₃): δ = 0,70-1,85 (40H), 2,22 (2H), 2,66 (1H), 2,78 (1H), 3,07 (1H), 4,64 (1H), 5,01 (1H), 5,09 (1H), 5,47 (1H), 5,53 (1H), 7,10-7,41 (9H), 7,49 (1H), 7,56 (1H) ppm.

### BEISPIEL 11

### [1R-[1α,2β(2R*,3S*),4α,4aβ,10aβ]]-3-[Acetylamino]-2-hydroxybenzenpropansäure-9-methylen-1,2,3,4,4a,9,10,10a-octahydro-1,12,12-trimethyl-4a,10a-epoxy-1,4-methanophenanthren-2-ylester

mg ( µmol) der nach Beispiel 11a dargestellten Verbindung setzt man in Analogie zu Beispiel 1 um und isoliert nach Aufarbeitung und Reinigung mg ( µmol, %) der Titelverbindung als farblosen Schaum.
¹H-NMR (CDCl₃): δ = 0,86 (3H), 0,93 (3H), 1,08 (3H), 1,74 (1H), 1,97 (3H), 2,35 (1H), 2,77 (1H), 2,83 (1H), 2,97 (1H), 3,09 (1H), 4,48 (1H), 4,97 (1H), 4,99 (1H), 5,52 (1H), 5,56 (1H), 7,19-7,42 (7H), 7,56 (2H), 7,61 (1H) ppm.

### BEISPIEL 11a

### [1R-[1α,2β(2R*,3S*),4α,4aβ,10aβ]]-3-[Acetylamino]-2-(triisopropylsilyloxy)benzenpropansäure-9-methylen-1,2,3,4,4a,9,10,10a-octahydro-1,12,12-trimethyl-4a,10a-epoxy-1,4-methanophenanthren-2-ylester

5,6 mg (20 µmol) der nach Beispiel 1b dargestellten und durch Kristallisation enantiomerenrein erhaltenen Verbindung A sowie 22 mg des nach Beispiel 1f dargestellten β-Lactams setzt man in Analogie zu Beispiel 1a um und isoliert nach Aufarbeitung 28 mg der Titelverbindung als Rohprodukt, das man ohne Reinigung weiter umsetzt.
¹H-NMR (CDCl₃): δ = 0,81 (3H), 0,88 (3H), 0,95-1,30 (25H), 1,38 (1H), 1,98 (3H), 2,14 (1H), 2,65 (1H), 2,80 (1H), 3,08 (1H), 4,63 (1H), 4,93 (1H), 4,99 (1H), 5,50 (1H), 5,56 (1H), 7,15-7,38 (4H), 7,42-7,58 (4H), 7,67 (1H) ppm.

## Patentansprüche

1. Borneolderivate der allgemeinen Formel I 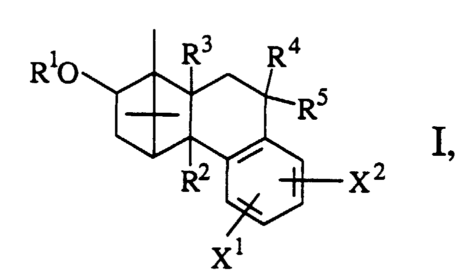 worin
R¹ C(O)-CH(OR⁶)-CH(NHR⁷)-R⁸,
R² Wasserstoff, -OH, C₁-C₁₀-Akyl, C₁-C₁₀-Alkoxy, -OC(O)R^{9a}, -OSO₂R^{9a}, -OP(O)(OH)₂, NHR^{9a}, NR^{9a}R^{9b},
R³ Wasserstoff, -OH, C₁-C₁₀-Alkoxy, -OC(O)R^{9b}, -OSO₂R^{9b}, -OP(O)(OH)₂, oder
R², R³ gemeinsam ein Sauerstoffatom,
R⁴ Wasserstoff, C₁-C₁₀-Alkyl, -(CH₂)ₙ-OR^{11a},
R⁵ Wasserstoff, C₁-C₁₀-Alkyl, -(CH₂)ₚ-OR^{11b}, oder
R⁴, R⁵ gemeinsam ein Sauerstoffatom, eine =CHR¹⁰-Gruppe,
n 0 bis 8
p 1 bis 8
R⁷ -C(O)R¹², -SO₂R¹², -C(O)OR¹², -C(O)NHR^{9d}, -C(O)NR^{9d}R^{9e}, 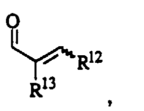
R⁸ Aryl,
R^{9a-e}, R¹² gleich oder verschieden sind und C₁-C₁₀-Alkyl, C₄-C₈-Cycloalkyl, Aryl, C₇-C₁₆-Aralkyl bedeuten,
R¹⁰ Wasserstoff, C₁-C₁₀-Alkyl, -(CH₂)_{S}-OR¹⁴,
s 1 bis 8
R⁶, R^{11a,b}, R¹⁴ gleich oder verschieden sind und Wasserstoff, C₁-C₁₀-Alkyl, Aryl, C₇-C₁₆-Ardkyl, -SO₂R^{9c}, -P(O)(OH)₂ bedeuten,
R¹³, R^{15a,b} gleich oder verschieden sind und Wasserstoff, C₁-C₁₀-Alkyl, Aryl, C₇-C₁₆-Aralkyl bedeuten,
X¹, X² gleich oder verschieden sind und X bedeuten,
X Wasserstoff, Halogen, -OH, -NO₂, -N₃, -CN, -NR^{15a}R^{15b}, -NHSO₂R^{15a}, -CO₂R¹⁵, C₁-C₁₀-Alkyl, C₁-C₁₀-Alkoxy, C₁-C₁₀-Acyloxy, C₁-C₁₀-Acyl sein kann
und, falls R¹⁵ Wasserstoff bedeutet, deren Salze mit physiologisch verträglichen Basen, sowie die α-, β- oder γ-Cyclodextrinclathrate, sowie die mit Liposomen verkapselten Verbindungen der Formel I bedeuten.

2. Arzneimittel, bestehend aus einer oder mehrerer Verbindungen des Anspruches 1 und üblicher Hilfs-, Träger- und Zusatzstoffe.

3. Verfahren zur Herstellung von Borneolderivaten der allgemeinen Formel I gemäß Patentanspruch 1, das dadurch gekennzeichnet, daß man ein Olefin der allgemeinen Formel II 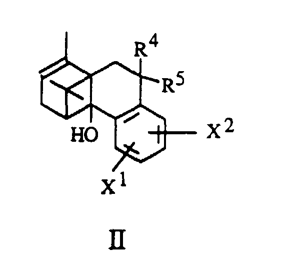 in dem R⁴, R⁵, X¹ und X² die oben genannten Bedeutungen haben und in X¹ oder X² enthaltene Hydroxylgruppen gegebenfalls geschützt sind, epoxidiert und das gebildete Epoxid ohne Isolation zu einem Alkohol der allgemeinen Formel III 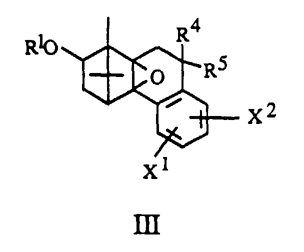 in dem R⁴, R⁵, X¹ und X² die oben genannten Bedeutungen haben und in R¹, X¹ oder X² enthaltene Hydroxylgruppen gegebenfalls geschützt sind, umlagert und dieses Umlagerungsprodukt in ein Derivat der allgemeinen Formel I überführt.

## Claims

1. Borneol derivatives of the general formula I 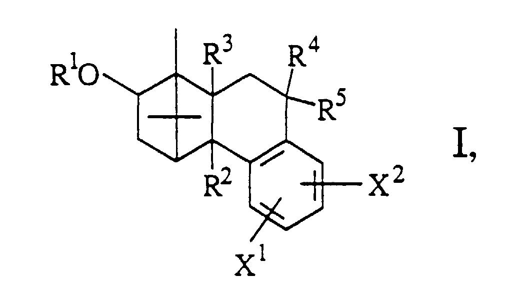 wherein
R¹ represents C(O)-CH(OR⁶)-CH(NHR⁷)-R⁸,
R² represents hydrogen, -OH, C₁-C₁₀alkyl, C₁-C₁₀alkoxy, -OC(O)R^{9a}, -OSO₂R^{9a}, -OP(O)(OH)₂, NHR^{9a} or NR^{9a}R^{9b},
R³ represents hydrogen, -OH, C₁-C₁₀alkoxy, -OC(O)R^{9b}, -OSO₂R^{9b} or -OP(O)(OH)₂, or R² and R³ together represent an oxygen atom,
R⁴ represents hydrogen, C₁-C₁₀alkyl or -(CH₂)ₙ-OR^{11a}
R⁵ represents hydrogen, C₁-C₁₀alkyl or -(CH₂)ₚ-OR^{11b}, or
R⁴ and R⁵ together represent an oxygen atom or a =CHR¹⁰ group,
n is from 0 to 8,
p is from 1 to 8,
R⁷ represents -C(O)R¹², -SO₂R¹², -C(O)OR¹², -C(O)NHR^{9d}, -C(O)NR^{9d}R^{9e} or 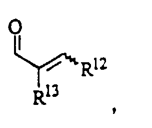
R⁸ represents aryl,
R^{9a-e} and R¹² are identical or different and each represent C₁-C₁₀alkyl, C₄-C₈cycloalkyl, aryl or C₇-C₁₆aralkyl.
R¹⁰ represents hydrogen, C₁-C₁₀alkyl or -(CH₂)ₛ-OR¹⁴,
s is from 1 to 8,
R⁶, R^{11a,b} and R¹⁴ are identical or different and each represent hydrogen, C₁-C₁₀alkyl, aryl, C₇-C₁₆aralkyl, -SO₂R^{9c} or -P(O)(OH)₂,
R¹³ and R^{15a,b} are identical or different and each represent hydrogen, C₁-C₁₀alkyl, aryl or C₇-C₁₆aralkyl,
X¹ and X² are identical or different and each represent X, and
X may be hydrogen, halogen, -OH, -NO₂, -N₃, -CN, -NR^{15a}R^{15b}, -NHSO₂R^{15a}, -CO₂R¹⁵. C₁-C₁₀alkyl, C₁-C₁₀alkoxy, C₁-C₁₀acyloxy or C₁-C₁₀acyl
and, if R¹⁵ represents hydrogen, salts thereof with physiologically tolerable bases, and α-, β-or γ-cyclodextrin clathrates thereof, and also compounds of formula I incorporated in liposomes.

2. Medicament consisting of one or more compounds of claim 1 and customary excipients. carriers and additives.

3. A process for the preparation of borneol derivatives of the general formula I according to patent claim 1, characterised in that an olefin of the general formula II 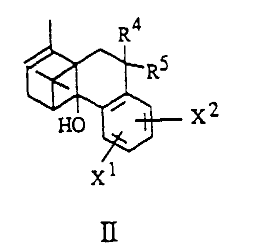 wherein R⁴, R⁵, X¹ and X² are as defined above and hydroxy groups present in X¹ or X² are optionally protected, is epoxidised and the resulting epoxide, without being isolated, is rearranged to form an alcohol of the general formula III 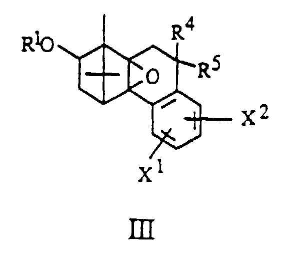 wherein R⁴, R⁵, X¹ and X² are as defined above and hydroxy groups present in R¹, X¹ or X² are optionally protected, and that rearrangement product is converted into a derivative of the general formula I.

## Revendications

1. Dérivés de bornéol de la formule générale 1 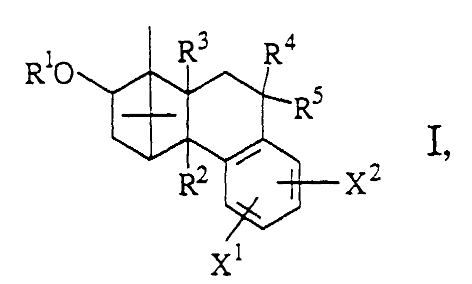 dans laquelle
R¹ représente C(O)-CH(OR⁶)-CH(NHR⁷)-R⁸,
R² représente de l'hydrogène, OH, un alkyle en C₁-C₁₀, un alcoxy en C₁-C₁₀, -OC(O)R^{9a}, -OSO₂R^{9a}, -OP(O)(OH)_{2,} NHR^{9a}, NR^{9a}R^{9b}_{,}
R³ représente de l'hydrogène, OH, un alcoxy en C₁-C₁₀, -OC(O)R^{9b}_{,} -OSO₂R^{9b}, -OP(O)(OH)₂,
R² et R³ pouvant former conjointement un atome d'oxygène,
R⁴ représente de l'hydrogène, un alkyle en C₁-C₁₀, -(CH₂)ₙ-OR^{11a}_{,}
R⁵ représente de l'hydrogène, un alkyle en C₁-C₁₀, -(CH₂)ₚ-OR^{11b}_{,}
R⁴ et R⁵ pouvant former conjointement un atome d'oxygène ou un groupe =CHR¹⁰,
n a une valeur de 0 à 8,
p a une valeur de 1 à 8,
R⁷ représente -C(O)R¹², -SO₂R¹², -C(O)OR¹², -C(O)NHR^{9d}, -C(O)NR^{9d}R^{9e}, 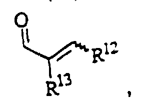
R⁸ représente un aryle,
R^{9a-e} et R¹² sont identiques ou différents et représentent un alkyle en C₁-C₁₀, un cycloalkyle en C₄-C₈, un aryle, un aralkyle en C₇-C₁₆,
R¹⁰ représente de l'hydrogène, un alkyle en C₁-C₁₀, -(CH₂)ₛ-OR¹⁴,
s a une valeur de 1 à 8,
R⁶, R^{11a,b} et R¹⁴ sont identiques ou différents et représentent de l'hydrogène, un alkyle en C₁-C₁₀, un aryle, un aralkyle en C₇-C₁₆, -SO₂R^{9c}, -P(O)(OH)₂,
R¹³ et R^{15a,b} sont identiques ou différents et représentent de l'hydrogène, un alkyle en C₁-C₁₀, un aryle, un aralkyle en C₇-C₁₆,
X¹ et X² sont identiques ou différents et représentent X,
X représente de l'hydrogène, un halogène, -OH, -NO₂, -N₃, -CN, -NR^{15a}R^{15b}, -NHSO₂R^{15a}, -CO₂R¹⁵, un alkyle en C₁-C₁₀, un alcoxy en C₁-C₁₀, un acyloxy en C₁-C₁₀ ou un acyle en C₁-C₁₀,
et, dans le cas où R¹⁵ représente de l'hydrogène, leurs sels avec des bases physiologiquement compatibles, ainsi que les α-, β- ou γ-cyclodextrineclathrates, ainsi que les composés de la formule I encapsulés par des liposomes.

2. Médicaments constitués d'un ou de plusieurs composés de la revendication 1 et d'adjuvants, supports et additifs courants.

3. Procédé de préparation de dérivés de bornéol de la formule générale I suivant la revendication 1, caractérisé en ce qu'on époxyde une oléfine de la formule générale Il 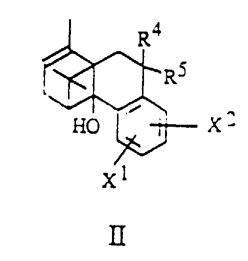 dans laquelle R⁴, R⁵, X¹ et X² ont les significations indiquées ci-dessus et des groupes hydroxyle contenus dans X¹ ou X² sont éventuellement protégés, et on transpose l'époxyde formé, sans isolement, en un alcool de la formule générale III 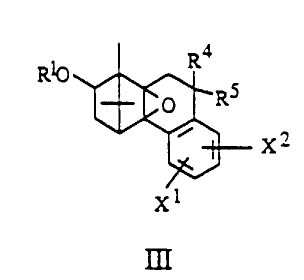 dans laquelle R⁴, R⁵, X¹ et X² ont les significations indiquées ci-dessus et des groupes hydroxyle contenus dans R¹, X¹ ou X² sont éventuellement protégés, et on convertit ce produit de transposition en un dérivé de la formule générale I.
